# EUROPEAN PATENT SPECIFICATION

(11) **EP 2 364 637 B1**
(45) Date of publication and mention of the grant of the patent: **18.07.2018**
(21) Application number: 11152091.2
(22) Date of filing: 25.01.2011
(51) Int. Cl.: A61B 1/01, A61B 1/015, A61M 25/06, A61B 1/31, A61M 25/04, A61M 25/10

(54) **An overtube with a balloon and an endoscope system**
Überrohr mit einem Ballon und einem Endoskopsystem
Sur-tube avec ballonnet et système d'endoscope

(30) Priority: 12.03.2010 JP 2010055348
(43) Date of publication of application: 14.09.2011
(73) Proprietor: FUJIFILM Corporation, Tokyo (JP)
(72) Inventor: Ueda, Yoshihiro, Saitama (JP)
(74) Representative: Klunker IP Patentanwälte PartG mbB

(56) References cited:
- EP-A2- 2 016 914
- US-A- 5 152 277
- US-A1- 2007 244 359
- US-A1- 2008 097 292

## Description

### FIELD OF THE INVENTION

The present invention relates to an overtube with a balloon attached to an inserter of an endoscope, for inserting the inserter into a deeper part of a body cavity, and relates to an endoscope system provided therewith.

### BACKGROUND OF THE INVENTION

In a medical field, an endoscope has been widely used for observing, diagnosing and treating inside a body cavity, for example a wall surface in a deep alimentary canal such as a small intestine and a large intestine.

Simply pushing the inserter, in many cases will not transfer the force to the tip of the inserter because of complex bends of the alimentary canal, making deep insertion difficult.

To address this problem, there has been proposed a method that the inserter of the endoscope is set in a tubular-shaped insert assist tool (also referred to as an overtube or a sliding tube) and the inserter is inserted into a body with the insert assist tool. According to the method, the inserter of the endoscope is guided by the insert assist tool to prevent the inserter from being bent or flexed more than necessary, so that the inserter can be inserted into the deeper part of the alimentary canal.

Japanese Patent Application Laid-Open No. 2009-022444 describes an overtube attached with a balloon at the tip thereof. According to the overtube with the balloon, the overtube can be fixed to the alimentary canal by inflating the balloon. The inserter and the overtube are alternately inserted while repeatedly inflating and deflating the balloon, and then the inserter of the endoscope can be smoothly inserted into the deeper part of alimentary canal.

In the overtube with the balloon, the overtube is moved in a removal direction in a manner that the balloon is tightly fixed to the inner wall of the canal with the balloon inflated, and the alimentary canal is pulled (folded) toward operator's hand. In this case, the problem arises in that air accumulated at rear of the balloon is compressed to increase air pressure, and the increased air pressure becomes resistance to the moving operation of the overtube in the removal direction, which makes difficult to fold the alimentary canal, resulting in a trouble of the insert operation of the endoscope.

In order to dissolve the problem, a first embodiment of the Japanese Patent Application Laid-Open No. 2009-022444 discloses an air hole that is provided on a peripheral surface of the overtube near the rear of the balloon. And, a connection path is formed in a tube wall of the overtube, for connecting the air hole and a connecter provided at a base end portion (holder) of the overtube. A control handle of the endoscope is provided with a connecter that is connected to the base end connecter of the overtube through a tube, and also provided with a suction tube which reaches an end ring of a light source connecter through the connecter, the control handle, and a universal code. A suction device is driven by connecting to the end ring of the light source, and air in the tube is suctioned from the air hole at nearest rear of the balloon and the air pressure increase at the rear of the balloon is controlled.

In a second embodiment of the Japanese Patent Application Laid-Open No. 2009-022444, the suction tube used in the first embodiment is connected to a forceps tube near the control handle. According to this structure, when a suction button provided to the endoscope is operated, the air at the rear of the balloon is exhausted through the air hole and the connection path of the overtube, and the forceps tube of the endoscope.

Moreover, the Japanese Patent Application Laid-Open No. 2009-022444 discloses that the tube that is connected between the base end of the overtube and the connecter of the control handle may be connected to forceps openings (in this case, two forceps openings are provided for the tube and a medical instrument) in place of providing the connecter at the control handle of the endoscope. It is described that the air hole may be provided on a tip surface of the overtube at nearest front of the balloon.

In the Japanese Patent Application Laid-Open No. 2009-022444, each embodiment requires an additional tube or modification of the endoscope. That is, it is necessary to provide the tube for connecting the connection path of the air hole and the forceps tube, the connecter of the control handle of the endoscope, and the tube for connecting the connecter and the forceps tube and like, so that the cost increases. Even when the tube that is connected between the base end of the overtube and the connecter of the control handle may be connected to forceps openings in place of providing the connecter at the control handle of the endoscope, two forceps openings are required, so that modification of the endoscope is required. Therefore, it is necessary for the medical facility to request modification of the existing endoscope to a vendor or buy a new endoscope, so that a capital-investment spending increases. An overtube as set out in the preamble of claim 1 is disclosed in US 5 152 277 A.

### SUMMARY OF THE INVENTION

An object of the present invention is to increase an operability of an overtube with balloon without adding an extra parts and modifying an endoscope.

To achieve the above object, an overtube with a balloon includes at least a first air hole and a second air hole located at front and rear of the balloon, wherein the first and second air holes connect each other to allow air flow between a front side and a rear side in a body cavity walled by the balloon. The over tube includes a body attached to a periphery of an inserter of an endoscope and the balloon is mounted on a tip of the body. The inserter is inserted into a body cavity while inflating and deflating the balloon.

The body is formed with an accommodation hole into which the inserter is inserted, and an area between the first hole and the second hole in a gap between the inserter and the accommodation hole forms a connection path for connecting the first hole and the second hole. The accommodation hole is closed at a tip and an end of the body. A taper is entirely or partly formed at periphery of the tip of the body so as to contact the periphery of the inserter, to entirely or partly close the accommodation hole.

The body is preferably formed with a connection path for connecting the first air hole and second air hole. Check valves are preferably provided to at least one of the first air hole and second air hole or provided to the connection path for allowing air flow from the rear side to the front side in the body cavity and blocking air flow from the front side to the rear side in the body cavity.

In an endoscope system of the present invention includes an endoscope where an inserter is inserted into a body cavity and an overtube with a balloon, the overtube with a balloon includes a body attached to a periphery of the inserter and a balloon mounted on a tip of the body. The inserter is inserted into the body cavity while inflating and deflating the balloon. The overtube includes at least first air hole and second air hole located at front and rear of the balloon and formed to the body, and the first and second air holes connect each other for allowing air flow between the front side and the rear side in the body cavity walled by the balloon.

The endoscope system further includes a forceps tube provided in the inserter, a forceps outlet port formed on a tip face of the inserter and located at an edge of the tube, and a suction device connected to the forceps tube. In the suction device, the air is suctioned from the forceps outlet port through the forceps tube and the air at the rear side in the body cavity is exhausted to the outside of the body cavity through the first and second air holes.

In the endoscope system, a balloon control device for supplying fluid to the balloon and suctioning the fluid from the balloon is provided for inflating and deflating the balloon.

The compressed air accumulated at the rear side of the balloon can by easily supplied to the front side of the balloon by providing the air holes at the both sides of the balloon and pulling the intestines canal in the body. Therefore, operability of the overtube with the balloon can be increased without adding extra parts and modifying the endoscope.

### BRIEF DESCRIPTION OF THE DRAWINGS

The above objects and advantages of the present invention will become easily understood by one of ordinary skill in the art when the following detailed description would be read in connection with the accompanying drawings.
Fig.1 is a schematic diagram showing an endoscope system;
Fig.2 is a plane view showing a tip surface of a rigid tip section of an endoscope;
Fig. 3 is a schematic diagram showing a pipe structure of the endoscope system;
Fig. 4 is an enlarged sectional view showing a tip of an overtube with a balloon according to a first embodiment;
Fig. 5 is an explanatory drawing showing a state that the overtube with the balloon is inserted into an intestine canal;
Fig.6 is an enlarged sectional view showing the tip of the overtube with the balloon according to a second embodiment; and
Fig. 7 is an enlarged sectional view showing an example that check valves are provided to air holes.

### DESCRIPTION OF THE PREFERRED EMBODIMENTS

### [First embodiment]

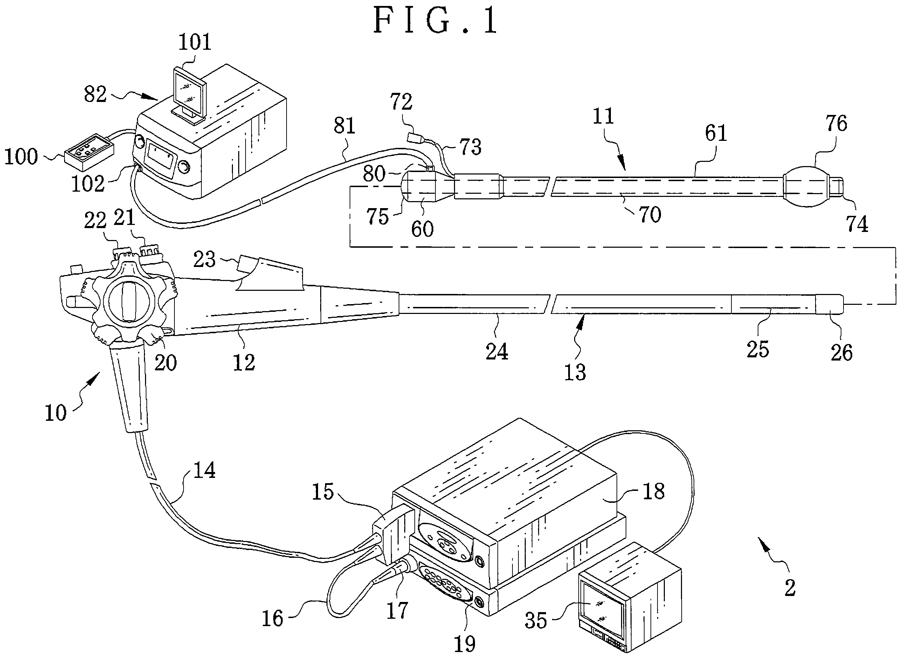

In Fig. 1, an endoscope system 2 is provided with an electronic endoscope 10 and an overtube with a balloon 11 (hereinafter referred to as an overtube). The electronic endoscope 10 has a control handle 12 and an inserter 13 that is connected to the control handle 12 and inserted into a body cavity, for example a large intestine. A universal code 14 is connected to the control handle 12 and a tip of the universal code 14 is provided with a connecter for light source 15. A cable 16 diverges from the connecter for light source 15 and a tip of the cable 16 is provided with a connecter for processor device 17. The connecter for light source 15 and the connecter for processor device 17 are detachably connected to a light source device 18 and a processor device 19 respectively.

The control handle 12 is provided with an angle knob 20, an air/water feed button 21 for discharging air or water from a tip of the inserter 13, and a suction button 22 and like. Moreover, a forceps channel 23 into which a medical instrument such as an electrosurgical knife is inserted, is provided to the inserter 13 side of the control handle 12.

The inserter 13 includes a flexible section 24 having flexibility, a bending section 25 and a rigid tip section 26, in the order from the control handle 12 side.

The flexible section 24 is a few meters long in order for the rigid tip section 26 to reach the intended position inside of the body. The bending section 25 bends in vertical and horizontal directions in cooperation with the operation of the angle knob 20 of the control handle 12. Thereby, the rigid tip section 26 can be oriented toward a desired direction.

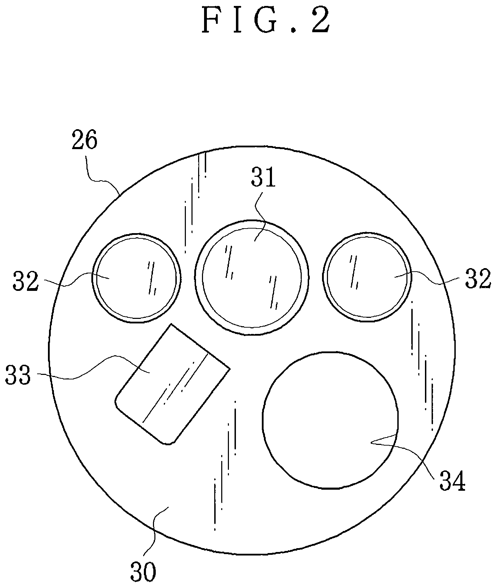

Shown in Fig. 2, a tip surface 30 of the rigid tip section 26 is provided with an imaging window 31, illumination windows 32, an air/water feed nozzle 33 and a forceps outlet port 34. The imaging window 31 is located one-side center of the tip surface 30. Two illumination windows 32 are located both sides of the imaging window 31.

An objective optical system for retrieving an image of an observed region in the body, and an image pickup device such as a CCD and a CMOS image sensor for taking the image of the observed region are provided behind the imaging window 31. The imaging pickup device is connected to the processor device 19 by a signal cable that reaches the connecter for processor device 17 through the inserter 13, the control handle 12 and the universal code 14. The image of the observed region retrieved from the imaging window 31 is focused on a light receiving surface of the imaging pickup device and converted into an imaging signal. The processor device 19 performs various image processing operations to the imaging signal received from the imaging pickup device via the signal cable, and converts it into a video signal to display it on a cable-connected monitor 35 (see Fig. 1) as an observed image.

An exit end of a light guide that leads illumination light from an illumination light source of the light source device 18 is disposed at back of the illumination window 32. The light guide reaches the connector for light source 15 through the inserter 13, the control handle 12 and universal code 14, and an entry end of the light guide is disposed in the connecter for light source 15. Illumination light guided by the light guide illuminates to the observed part in the body via the illumination window 32.

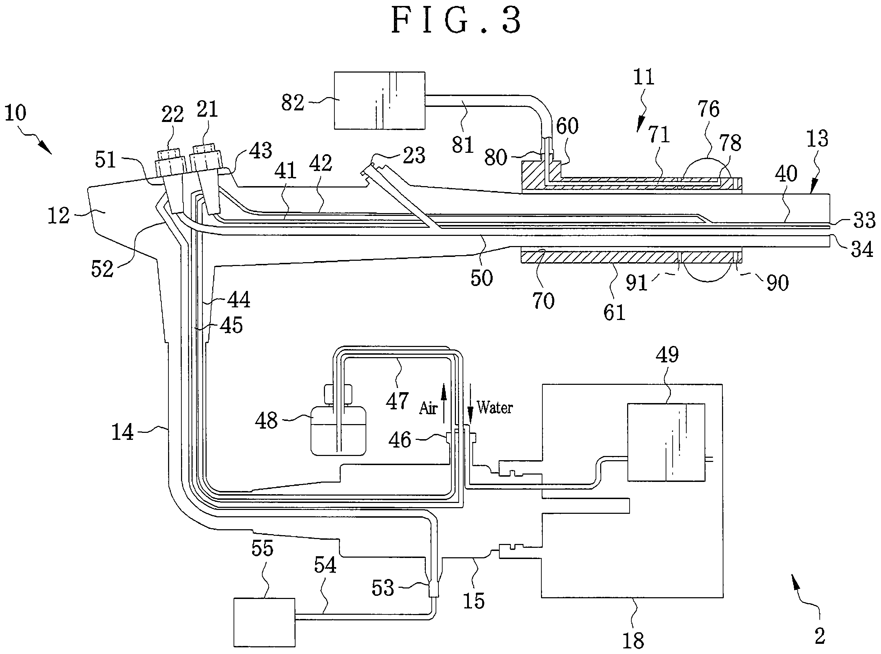

In Fig. 3, an air/water feed tube 40 is connected to the air/water feed nozzle 33. The air/feed tube 40 divides into an air feed tube 41 and water feed tube 42, and they are respectively connected to a bulb 43 disposed at the control handle 12. An air feed tube 44 and a water feed tube 45 are connected to the bulb 43, and the air/water feed button 21 is attached to the bulbs 43. The air/water feed button 21 is formed with an air hole (not shown) for connecting the aeration tube 44 to open air.

The air feed tube 41 and the air feed tube 44 are connected in a state that the air/water feed button 21 protrudes. However, since the air hole formed at the center of the air/water feed button 21 opens, air supplied from the air feed tube 44 is not supplied to the air feed tube 41. When the air hole of the air/water feed button 21 is covered by the operator, air supplied from the air feed tube 44 is supplied to the air feed tube 41. When the air/water feed button 21 is pushed down, the water feed tube 42 and the water feed tube 45 are connected and water is discharged from the air/water feed nozzle 33.

The air feed tube 44 and the water feed tube 45 is inserted through the universal code 14 and reaches a water feed end ring 46 of the connecter for light source 15. A tube 47 is detachably connected to the water feed end ring 46, in which a tip of the tube 47 is connected to a water tank 48. A tip of the air feed tube 44 is connected above the water impounded in the water tank 48 and a tip of the water feed tube 45 is connected below the water.

The air feed tube 44 is divided in the water feed end ring 46 and the divided tip of the air feed tube 44 is connected to an air pump 49 in the light source device 18. The air pump 49 is driven to supply air, and the air is supplied to the air feed tube 44. The supplied air is exhausted exterior through an air hole of the suction button 21 in a manner that the suction button 21 is protruded. When the operator covers the air hole, the supplied air is exhausted toward the imaging window 31 from the air/water feed nozzle 33 through the air feed tube 41 and the like.

When the air/water feed button 21 is pressed down, the connection between the air feed tube 44 and the air feed tube 41 is cut off. Therefore, the air supplied to the air feed tube 44 is supplied above water surface in the water tank 48. Accordingly, inner pressure of the water tank 48 increases to supply water to the water feed tube 45, and the water is discharged toward the imaging window 31 from the air/water feed nozzle 33 through the water tube 42.

A forceps tube 50 is connected to the forceps outlet port 34. The forceps tube 50 is divided in the inserter 13, and the divided tubes are respectively connected to the forceps channel 23 and a bulb 51. A medical instrument is inserted from the forceps channel 23 and a tip of the medical instrument can be led out from the forceps outlet port 34.

A suction tube 52 is connected to the bulb 51 and the suction button 22 is attached to the bulb 51. The suction tube 52 is connected to open air in a manner that the suction button 22 is protruded. When the suction button 22 is pressed down, the suction tube 52 and the forceps tube 50 are connected. The suction tube 52 reaches a suction end ring 53 of the connecter for light source 15. A tube 54 is detachably connected to the suction end ring 53 and connected to a suction device 55. When the suction device 55 is driven and the suction device 22 is pressed down, air and the lesion inside of the body can be suctioned from the forceps outlet port 34.

The overtube 11 includes a holder 60 grasped by a doctor and a body 61 (also see Fig.1). The holder 60 is a tubular rigid material such as plastic. The body 61 is formed in an almost tubular shape made of a flexible material such as polyurethane and fit onto a tip of the holder 60.

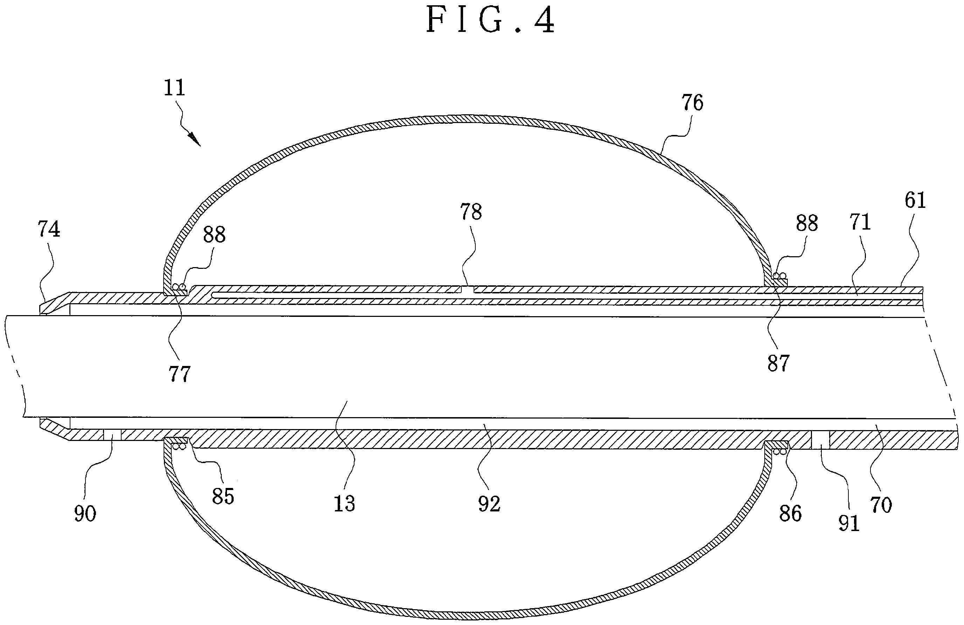

Shown in Fig. 4, an inside of the body 61 is formed with an accommodation hole 70 and a flow conduit for balloon 71 in an axis direction thereof. The accommodation hole 70 is for inserting the inserter 13 of the electronic endoscope 10. The accommodation hole 70 is a round shape in its cross sectional surface that intersects to the axis direction of the body 61, in which an inner diameter of the accommodation hole 70 is larger than an outer diameter of the inserter 13 and a gap is formed between the periphery of the inserter 13 and the inner surface of the accommodation hole 70. Hydrophile coat layer (lubricating coat layer) such as polyvinylpyrrolidone is coated on an inner peripheral surface of the accommodation hole 70.

When using the overtube 11, lubricant such as water is supplied to an inner peripheral surface of the accommodation hole 70 (a gap between the inserter 13 and the body 61) to decrease friction between the inserter 13 and the body 61. The lubricant is injected by a syringe or the like (not shown) from a connecter 72 shown in Fig. 1. The connecter 72 is connected to a small diameter tube 73, where a tip of the tube 73 is connected to a base end of the accommodation hole 70. The lubricant injected from the connector 72 is supplied to the inner peripheral surface of the accommodation hole 70 through the tube 73.

A tip of the body 61 is formed with a taper 74 for narrowing the inner diameter of the body 61 (also see Fig. 1). The taper 74 enables to close a gap between the inserter 13 and the tip of the body 61 when the inserter 13 of the electronic endoscope 10 is inserted through the accommodation hole 70, thereby leakage of lubricant to the tip side of the body 61 is prevented. Similarly, in order to prevent leakage of the lubricant, a tube 75 is provided at a base end of the overtube 11 (at a base end of the holder 60) (see Fig.1). The tube 75 is made of an elastic material such as a rubber and whose diameter is formed gradually narrow to the base end side. In a similar manner to the taper 74, the small diameter of the tube 75 prevents leakage of lubricant to the exterior. It is noted that the taper 74 may be provided to all circumference of the tip of the body 61 or may be partly formed in the outer circumference.

The flow conduit for balloon 71 is a conduit for supplying and suctioning fluid (for example, air) to and from a balloon 76 and it is formed in the body 61. The flow conduit for balloon 71 is formed in an oval shape whose sectional shape that intersects in an axial direction of the body 61 is short in a radial direction of the body 61 and long in a circumferential direction. Therefore, it is possible to control protrusion of the flow conduit for balloon 71 of the body 61 with sufficiently keeping the flowing area of the flow conduit for balloon 71. It is noted that the flow conduit for balloon 71 may have a cross-sectional shape which is short in a radial direction and long in a circumferential direction. For example, the flow conduit for balloon 71 may be a flat shape that curves parallel to the inner peripheral face of the accommodation hole 70.

The tip of the flow conduit for balloon 71 ends at a fixing position of an leading end portion 77 of the balloon 76. Moreover, the flow conduit for balloon 71 is connected to an opening for balloon 78 formed on the peripheral face of the body 61. The opening 78 is formed at the attach position of the balloon 76 (in particular, formed at the intermediate position between concave portions 85 and 86). Supply and suction of the air from the opening 78 allows inflation and deflation of the balloon 76.

In Figs 1 and 3, the base end of the flow conduit for balloon 71 is connected to the connector 80 of the holder 60. A tube 81 is connected to a connecter 80 and a balloon control device 82. The air is supplied and suctioned by the balloon control device 82 to inflate and deflate the balloon 76. Moreover, a small diameter tube may be connected to the base end of the flow conduit for balloon 71 in place of the connecter 72 and the tube 73, and the connecter 80 may be provided at the end of the tube.

In Fig. 4, a periphery face of the tip of the body 61 is formed with two concave portions 85 and 86 at predetermined intervals where the balloon 76 is mounted. The concave 85 at the tip side is provided through the whole circumference of the periphery face of the body 61. The concave 86 at the base end side is formed in a C-shape which surrounds the flow conduit for balloon 71. The leading end portion 77 and a base end portion 87 of the balloon 76 are respectively fixed to the concaves 85 and 86.

When the balloon 76 inflates, the balloon 76 becomes an almost spheroid shape whose central portion inflates. The end portion 77 of the balloon 76 covers the concave 85 of the body 61 in a manner that the balloon 76 is inside out. Then, a thread 88 is wound on the end portion 77 of the balloon 76 and glue is applied thereon to fix the leading end portion 77 of the balloon 76 to the body 61. Next, the balloon 76 is turned back from an inside out manner and the base end portion 87 of the balloon 76 is covered on the concave 86. And, the thread 88 is wound on the base end portion 87 of the balloon 76 and glue is applied thereon to fix the base end portion 87 of the balloon 76 to the body 61. Accordingly, the whole of the leading end portion 77 and the base end portion 87 of the balloon 76 are respectively fixed to the concaves 85 and 86. Since most of the leading end portion 77 and the base end portion 87 of the balloon 76 are disposed at concaves 85 and 86, the fixed portions of the balloon 76 is mostly fixed without protrusion.

A plurality of air holes 90 and 91 is formed on the periphery of the body 61, which are located at nearest front and rear of the balloon 76 (front and rear of the concaves 85 and 86) and also located at the different positions from the flow conduit for balloon 71. The air holes 90 and 91 are provided at regular intervals (for example 4 holes each 90 degrees) in a circumferential direction of the body 61, located around the low conduit for balloon 71, and passes through the accommodation hole 70 from the outer surface of the body 61. A connection path 92 for flowing the air from the rear hole 91 to the front air hole 90 is constituted from the air holes 90 and 91 and the accommodation hole 70 positioned between them. It is noted that plural air holes 90 and 91 may be arranged in an axial direction of the body 61.

The body 61 is manufactured by processing a multi lumen tubing having a uniform sectional shape. In the multi lumen tubing prior to processing operation, two holes for the accommodation hole 70 and the flow conduit for balloon 71 are formed passing through in an axial direction, in which the cross section perpendicular to the tube axis is uniformly formed. After a cored bar is inserted into the multi lumen tubing, the air holes 90 and 91 are made by a cylinder shaped punch. Next, a tubular mold having two convex portions on the inner periphery surface is pushed from the outside and heated at a predetermined temperature, for example 100 to 110 degrees Celsius, to form the concave portions 85 and 86, finally the body 61 is completed.

In Fig. 1, the balloon control device 82 is a device for supplying and suctioning fluid, for example air, to and from the balloon 76, and a control box 100 and a balloon monitor 101 are attached thereto. A front face of the balloon control device 82 is provided with a power source switch, a hold switch and a pressure display panel and so on. The pressure display panel is a panel for displaying pressure value of the balloon 76, in which error code is displayed on the pressure display panel when a trouble occurs, for example the balloon 76 is broken. A backflow prevention unit 102 is provided at a connection point of the tube 81 that supplies and suctions the air to and from the balloon 76 and the balloon control device 82. The backflow prevention unit 102 is configured by incorporating a vapor-liquid separation filter into a hollow-disk-shaped case detachably attached to the balloon control device 82. When the balloon 76 is broken, the filter prevents fluid such as body fluid from flowing in the balloon control device 82.

The control box 100 is provided with various switches. For example, a hold switch having same function as the hold switch of the balloon control device 82 and an ON/OFF switch for instructing pressurization/depressurization of the balloon 76 and a pause switch for holding pressure of the balloon 76. The control box 100 is electrically connected to the balloon control device 82 via a code. It is noted that the control box 100 is provided with a display panel displaying an air-supply state or an exhaust state of the balloon 76 (not shown).

The balloon control device 82 supplies the air to the balloon 76 to inflate it and controls the air pressure at a constant value to maintain the inflated state of the balloon 76. In addition, , the balloon control device 82 suctions the air from the balloon 76 to deflate it, and also controls the air pressure to a constant value to maintain the deflated state of the balloon 76.

The balloon monitor 101 displays the pressure value and the inflated/deflated state of the balloon 76 when the balloon 76 is inflated or deflated. It is noted that the pressure value and the inflated/deflated state of the balloon 76 may be displayed on a monitor 35 with being superimposed on the image observed by the endoscope 10.

Next, a method of operating the electronic endoscope system 2 configured above will be described.

In the case where the overtube 11 is used, the inserter 13 of the electronic endoscope 10 is inserted into a predetermined position in the accommodation hole 70 to combine each other. Next, a tube 81 is fitted in the connecter 81 to connect the balloon control device 82 and the overtube 11. Lubricant, for example water is injected into the connecter 72 to facilitate relative movement between the body 61 of the overtube 11 and the inserter 13 of the electric endoscope 10.

The inserter 13 is gently inserted into the body cavity in a state that the overtube 11 is attached to the inserter 13 of the electronic endoscope 10. The balloon control device 82 is operated as necessary during the insertion, and inserter 13 is further inserted into the deeper portion of the body cavity with repeatedly inflating and deflating the balloon 76.

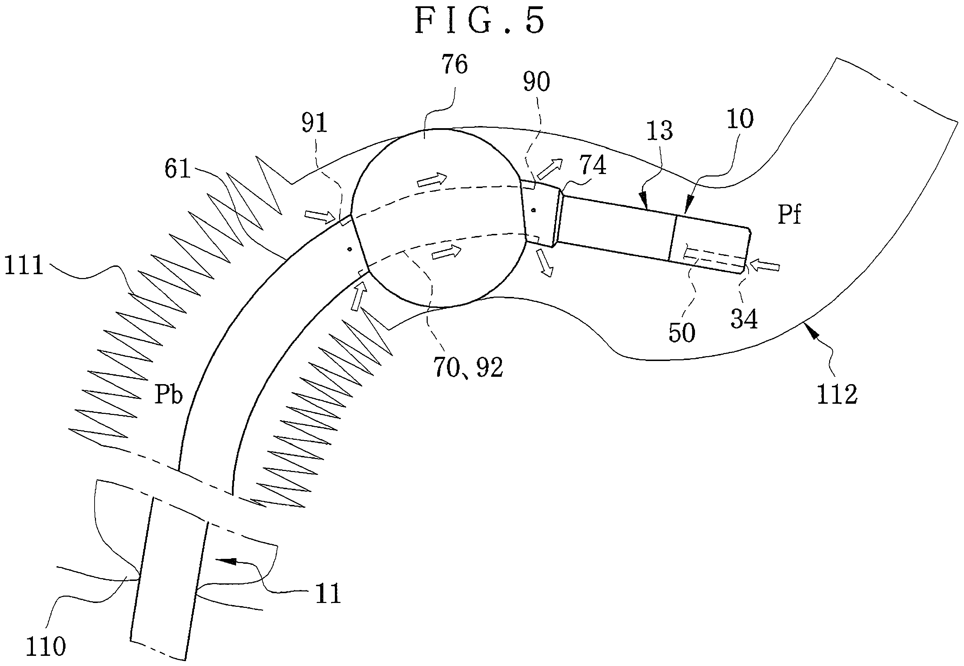

In particular, shown in Fig. 5, the inserter 13 of the electronic endoscope 10 is inserted from an anus 110. When a tip of the inserter passes through an S-shaped colon 111, the balloon 76 is inflated to fix the overtube 11 to an intestines canal 112. When the overtube 11 is pulled against the inserter 13, the S-shaped colon 111 is bended by the balloon 76 to maintain a linear shape. Next, the inserter 13 is inserted in the overtube 11 and the tip of the inserter 13 is inserted into the deeper part of the intestines canal 112.

After pressing the inserter 13 in the overtube 11, the air in the balloon 76 is exhausted to deflate the balloon 76. After the balloon 76 is deflated, the overtube 11 is slid toward the deeper part of the intestines canal 112 against the inserter 13, to return the overtube 11 to the original position. Hereinafter, the inserter 13 is inserted into the deeper part of the intestines canal 112, by repeatedly inflating the balloon 76, pulling the overtube 11, pushing the inserter 13, deflating the balloon 76, and inserting the overtube 11.

In the operation where the overtube 11 is moved in a removal direction in a state that the balloon 76 is inflated, the S-shaped colon 111 is pulled toward the anus 110 and folded up into accordion. Therefore, the air accumulated at the rear of the balloon 76 is pressed to increase air pressure Pb which becomes resistance to the removal operation of the overtube 11.

However, the air holes 90 and 91 are provided at nearest front and rear of the balloon 76, and the connection path 92 is formed with the air holes 90 and 91 and the accommodation hole 70 between the air holes 90 and 91. The air at rear of the balloon 76 whose air pressure Pb is increased by pulling the S-shaped colon 111 is exhausted to the front of the balloon 76 through the rear air hole 91, the connection path 92 and the front air hole 90 shown in an arrow.

Since the air at rear of the balloon 76 is exhausted to the front of the balloon 76 through the connection path 92, the air pressure Pf at front of the balloon 76 increases. However, the air at front of the balloon 76 is connected to the suction device 55 through the forceps outlet port 34, the forceps tube 50, the suction tube 52 and the tube54. When the suction button 22 is pushed down by the operator, the air at front of the balloon 76 is suctioned to decrease the air pressure Pf.

Therefore, when the overtube 11 is operated in the removal direction, the air accumulated between the body 61 at the rear of the balloon 76 and an intestinal wall is compressed, thereby a trouble in the removal operation can be prevented and the overtube 11 can be operated in a removal direction smoothly.

Moreover, the overtube 11 is simply provided with the air holes 90 and 91, and any improvement of the electronic endoscope 10 is not necessary, thereby the cost rise can be minimized and facility investment can be cut down.

It is not necessary to connect an additional tube to the electronic endoscope 10 and to the overtube 11 for reducing the air pressure Pb at the rear of the balloon 76, so that the parts cost can be reduced. Moreover, the tube is not impeditive during insert and removal operation of the overtube 11 and operability of the overtube 11 can be increased compared to the case that the additional tube is connected.

### [second embodiment]

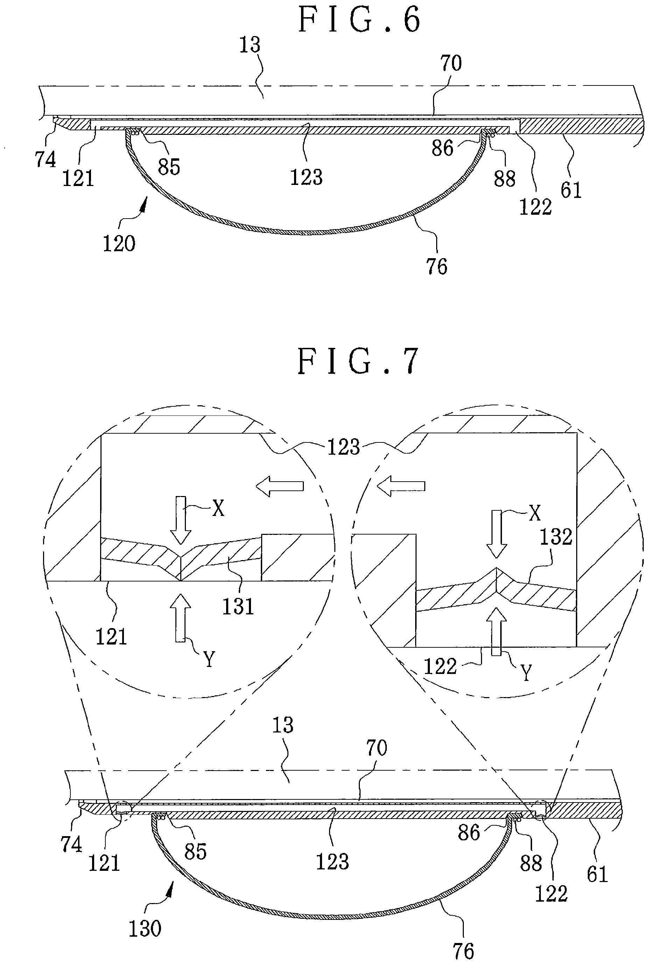

In the above first embodiment, the accommodation hole is also used as the connection path, but a dedicated connection path may be provided. In Fig.6, the body 61 of the overtube 120 has air holes 121 and 122 provided at front and rear of the balloon 76 and a connection path 123 separately provided from the accommodation hole 70 for communicating the air holes. The air holes 121 and 122 do not pass through the accommodation hole 70, so the accommodation hole 70 is not connected to the exterior. The same constituent elements as those of the first embodiment are designated with the same symbols, and explanation thereof will not be repeated.

Also in the above described structure, the air pressure Pb at the rear of the balloon 76 can be reduced as in the first embodiment, so that the insert and removal operation of the overtube 11 can be performed smoothly. Moreover, since the connection path 123 is provided separate from the accommodation hole 70, the air at the rear of the balloon 76 is always exhausted to front of the balloon 76 after passing through the connection path 123. Therefore, in the first embodiment where the accommodation hole 70 is used as the connection path 92, when the exhaust capacity of the air holes 90 and 91 and the connection path 92 is over, for example, a sudden increase of the air pressure Pb at the rear of the balloon 76, the air that fails to be supplied to the front of the balloon 76 is exhausted exterior from the base end of the holder 60 through the accommodation hole 70 with intestinal fluid and the surrounding may be contaminated, but the above anxiety is not necessary in the present embodiment. In order to prevent contamination in the first embodiment, a check valve for preventing exhausting air from the accommodation hole 70 is preferably provided at the holder 60.

In an overtube 130, check valves 131 and 132 are respectively provided at the air holes 121 and 122 shown in an enlarged view surround with two-dot chain line in Fig.7. For example, the check valves 131 and 132 are a duck bill type valve made of, for example, an elastic rubber.

The cheek valve 131 disposed at the air hole 121 at front of the balloon 76 allows air flow toward X-direction (air flow from the connection path 123 to the exterior through the air hole 121) and blocks air flow toward Y-direction (air flow from the exterior part to the connection path 123 through the air hole 121) . On the other hand, the cheek valve 132 disposed at the air hole 122 allows air flow toward Y direction (air flow from the exterior to the connection path 123 through the accommodation hole 122) and blocks air flow toward X-direction (air flow from the connection path 123 to the exterior through the air hole 122) . By the function of the check valves 131 and 132, the air once exhausted to the front of the balloon 76 from the rear thereof is never returned to the rear of balloon 76. It is possible to efficiently exhaust the air to the front of the balloon 76 from the rear thereof.

It is noted that the check valves 131 and 132 are provided at both of the holes 121 and 122. However, the connection path 123 may be provided at each of the check valves 131 and 132. Moreover, the check valves 131 and 132 may be provided at the holes 90 and 91 of the overtube 11 in Fig. 4.

In the above embodiments, the single balloon endoscope system in which the balloon is only attached to the overtube is explained. However, the present invention may be applied to a double balloon endoscope system that also includes an inflatable and deflatable balloon attached to the inserter of the electronic endoscope. In this case, it is possible to insert the tip of the inserter into the deep part of the intestines canal by repeatedly performing operation of inserting the inserter of the electronic endoscope, fixing the inserter by inflating the balloon at the inserter side, pushing the overtube along the inserter, holding the intestines canal by inflating the balloon of the overtube, and pulling the overtube. According to the present invention, the effect similar to the above is produced in the operation of pulling the obertube.

Various changes and modifications are possible in the present invention and may be understood to be within the present invention.

## Claims

1. An overtube (11) with a balloon (76) including a tubular body (61) attachable to a periphery of an inserter (13) of an endoscope (1) and a balloon (76) mounted on a periphery face of a tip of said body (61), said inserter (13) being insertable into a body cavity while inflating and deflating said balloon (76), said overtube (11) including at least a first air hole (90, 121) and a second air hole (91, 122) located at front and rear of said balloon (76), wherein the first air hole (90, 121) and the second air hole (91, 122) connect each other to allow air flow between front side and rear side in the body cavity walled by said balloon (76), **characterized in that** said body (61) is formed with an accommodation hole (70) where the inserter (13) is insertable, and an area between said first air hole (90) and said second air hole (91) in a gap between the periphery of said inserter (13) when inserted and the inner surface of said accommodation hole (70) forms a connection path (92) for connecting said first air hole (90) and said second air hole (91).

2. An overtube with a balloon according to claim 1, **characterized in that** said accommodation hole (70) is closed at a tip and an end of said body (61).

3. An overtube with a balloon according to claim 2, **characterized in that** a taper (74) is entirely or partly formed at a periphery of the tip of said body (61) so as to contact the periphery of said inserter (13), to entirely or partly close said accommodation hole (70).

4. An overtube with a balloon according to claim 1, **characterized in that** a check valve (131, 132) is provided to at least one of said first air hole (90, 121) and said second air hole (91, 122) or provided to said connection path (92), for allowing air flow from the rear side to the front side in said body cavity and blocking air flow from said front side to said rear side in said body cavity.

5. An endoscope system including an endoscope (1) that is provided with an inserter (13) insertable into a body cavity and including an overtube with a balloon (11) according to claim 1.

6. An endoscope system according to claim 5, **characterized by** comprising:
a forceps tube (50) provided in said inserter (13);
a forceps outlet port (34) formed on a tip face of said inserter (13) and located at an edge of said tube (50); and
a suction device (55) connected to said forceps tube (50), wherein air is suctioned from said forceps outlet port (34) through said forceps tube (50) and the air at the rear side in the body cavity is exhausted to the outside of said body cavity through said first air hole (90, 121) and said second air hole (91, 122).

7. An endoscope system according to claim 6, **characterized in that** a balloon control device (82) supplying and suctioning fluid to and from said balloon (76) is provided for inflating and deflating said balloon (76).

## Patentansprüche

1. Überrohr (11) mit einem Ballon (76), enthaltend einen schlauchförmigen Körper (61), der am Umfang eines Einführers (13) eines Endoskops (1) anbringbar ist, und einen an der Umfangsseite eines Endes des Körpers (61) angebrachten Ballon (76), wobei der Einführer (13) in einen Körperhohlraum einführbar ist, während der Ballon (76) aufgeblasen und abgelassen wird, wobei das Überrohr (11) mindestens ein erstes Luftloch (90, 121) und ein zweites Luftloch (91, 122) vor bzw. hinter dem Ballon (76) enthält, wobei das erste Luftloch (90, 121) und das zweite Luftloch (91, 122) miteinander in Verbindung stehen, damit ein Luftstrom zwischen der Frontseite und der Rückseite des von dem Ballon (76) abgesperrten Körperhohlraums möglich ist,
**dadurch gekennzeichnet, dass** der Körper (61) mit einem Aufnahmeloch (70) ausgebildet ist, in das der Einführer (13) einführbar ist, und ein Bereich zwischen dem ersten Luftloch (90) und dem zweiten Luftloch (91) in einer Lücke zwischen dem Umfang des eingeführten Einführers (13) und der Innenfläche des Aufnahmelochs (70) einen Verbindungsweg (92) zum Verbinden des ersten Luftlochs (90) mit dem zweiten Luftloch (91) bildet.

2. Überrohr mit einem Ballon nach Anspruch 1, **dadurch gekennzeichnet, dass** das Aufnahmeloch (70) am Vorderende und an einem Hinterende des Körpers verschlossen ist.

3. Überrohr mit Ballon nach Anspruch 2, **dadurch gekennzeichnet, dass** an einem Umfang des Endes des Körpers (61) teilweise oder vollständig eine Verjüngung (74) ausgebildet ist, um den Umfang des Einführers (13) zu berühren und damit das Aufnahmeloch (70) teilweise oder vollständig zu verschließen.

4. Überrohr mit Ballon nach Anspruch 1, **dadurch gekennzeichnet, dass** ein Rückschlagventil (131, 132) an mindestens einem von dem ersten Luftloch (90, 121) und dem zweiten Luftloch (91, 122) oder an dem Verbindungsweg (92) vorgesehen ist, um einen Luftstrom von der Rückseite zu der Vorderseite des Körperhohlraums zu ermöglichen und einen Luftstrom von der Vorderseite zu der Rückseite in dem Körperhohlraum zu sperren.

5. Endoskopsystem mit einem Endoskop (1), das mit einem in einem Körperhohlraum einführbaren Einführer (13) ausgestattet ist und ein Überrohr mit Ballon (11) gemäß Anspruch 1 enthält.

6. Endoskopsystem nach Anspruch 5, **gekennzeichnet durch**:
einen Forcepsschlauch (50), der in dem Einführer (13) vorgesehen ist;
eine Forceps-Auslassöffnung (34), die an einer Stirnseite des Einführers (13) ausgebildet ist und sich an einem Rand des Schlauchs (50) befindet; und
eine Saugeinrichtung (55), die an den Forcepsschlauch (50) angeschlossen ist, wobei durch den Forcepsschlauch (50) Luft aus der Forceps-Auslassöffnung (34) gesaugt wird und die Luft an der rückwärtigen Seite des Körperhohlraums durch das erste Luftloch (90, 121) und das zweite Luftloch (91, 122) aus dem Körperhohlraum ausgeleitet wird.

7. Endoskopsystem nach Anspruch 6, **dadurch gekennzeichnet, dass** eine Ballonsteuereinrichtung (82), die Fluid in den Ballon (76) einbringt und aus diesem absaugt, vorgesehen ist, um den Ballon (76) aufzublasen und abzulassen.

## Revendications

1. Surtube (11) doté d'un ballonnet (76) incluant un corps tubulaire (61) pouvant être fixé sur une périphérie d'un moyen d'introduction (13) d'un endoscope (1) et un ballonnet (76) monté sur une face de périphérie d'une pointe dudit corps (61), ledit moyen d'introduction (13) pouvant être introduit dans une cavité de corps tout en gonflant et dégonflant ledit ballonnet (76), ledit surtube (11) incluant au moins un premier évent (90, 121) et un second évent (91, 122) situés devant ou derrière ledit ballonnet (76), dans lequel le premier évent (90, 121) et le second évent (91, 122) sont reliés l'un à l'autre pour permettre une circulation d'air entre le côté avant et le côté arrière dans la cavité de corps cloisonnée par ledit ballonnet (76), **caractérisé en ce que** ledit corps (61) est formé d'un trou de logement (70) où le moyen d'introduction (13) peut être introduit, et une zone entre ledit premier évent (90) et ledit second évent (91) dans un espace entre la périphérie dudit moyen d'introduction (13), en situation d'introduction, et la surface intérieure dudit trou de logement (70) forme un trajet de liaison (92) pour relier ledit premier évent (90) et ledit second évent (91).

2. Surtube doté d'un ballonnet selon la revendication 1, **caractérisé en ce que** ledit trou de logement (70) est fermé sur une pointe et une extrémité dudit corps (61).

3. Surtube doté d'un ballonnet selon la revendication 2, **caractérisé en ce qu'**une diminution progressive (74) est entièrement ou partiellement formée sur une périphérie de la pointe dudit corps (61) pour un contact avec la périphérie dudit moyen d'introduction (13), de manière à fermer entièrement ou partiellement ledit trou de logement (70).

4. Surtube doté d'un ballonnet selon la revendication 1, **caractérisé en ce qu'**un clapet de non-retour (131, 132) est prévu sur au moins un desdits premier évent (90, 121) et second évent (91, 122), ou prévu sur ledit trajet de liaison (92), pour permettre une circulation d'air du côté arrière au côté avant dans ladite cavité de corps, et pour bloquer la circulation d'air dudit côté avant audit côté arrière dans ladite cavité de corps.

5. Système endoscopique incluant un endoscope (1) doté d'un moyen d'introduction (13) pouvant être introduit dans une cavité de corps et incluant un surtube doté d'un ballonnet (11) selon la revendication 1.

6. Système endoscopique selon la revendication 5, **caractérisé en ce qu'**il comprend :
un tube de pince (50) prévu dans ledit moyen d'introduction (13) ;
un orifice de sortie de pince (34) formé sur une face de pointe dudit moyen d'introduction (13) et situé sur un bord dudit tube (50), et
un dispositif d'aspiration (55) relié audit tube de pince (50), dans lequel l'air est aspiré à partir dudit orifice de sortie de pince (34) à travers le tube de pince (50), et l'air sur le côté arrière dans la cavité de corps est extrait vers l'extérieur de ladite cavité de corps à travers ledit premier évent (90, 121) et ledit second évent (91, 122).

7. Système endoscopique selon la revendication 6, **caractérisé en ce qu'**un dispositif de commande de ballonnet (82) alimentant et aspirant un fluide en direction et en provenance dudit ballonnet (76) est prévu pour gonfler et dégonfler ledit ballonnet (76).
